(19) Europäisches Patentamt

European Patent Office

Office européen des brevets

(11) **EP 4 467 125 A2**

(12) **EUROPEAN PATENT APPLICATION**

(43) Date of publication:
**27.11.2024 Bulletin 2024/48**

(21) Application number: **24206486.3**

(22) Date of filing: **31.07.2020**

(51) International Patent Classification (IPC):
***A61K 6/822*** *(2020.01)*

(52) Cooperative Patent Classification (CPC):
**A61K 6/818; A61K 6/822; C04B 35/486;**
C04B 2235/3225; C04B 2235/6562;
C04B 2235/6565; C04B 2235/6567; C04B 2235/96;
C04B 2235/9661

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.08.2019 JP 2019142362**

(62) Document number(s) of the earlier application(s) in
accordance with Art. 76 EPC:
**20848494.9 / 4 008 699**

(71) Applicant: **Kuraray Noritake Dental Inc.
Kurashiki-shi, Okayama 710-0801 (JP)**

(72) Inventors:
• **SAKAMOTO, Hiroyuki
Miyoshi-shi,, 470-0293 (JP)**
• **KATO, Shinichiro
Miyoshi-shi, 470-0293 (JP)**
• **MATSUURA, Atsushi
Miyoshi-shi, 470-0293 (JP)**

(74) Representative: **D Young & Co LLP
3 Noble Street
London EC2V 7BQ (GB)**

Remarks:
This application was filed on 14.10.2024 as a
divisional application to the application mentioned
under INID code 62.

(54) **METHOD FOR PRODUCING ZIRCONIA SINTERED BODY**

(57) The present invention provides a method that is for producing a zirconia sintered body and by which a zirconia molded body or a zirconia pre-sintered body is sintered in a short period of time, the zirconia sintered body reproducing an aesthetic requirement and strength of an ideal dental prosthesis at the same levels as those of a zirconia sintered body obtained by general firing. The present invention relates to a method for producing a zirconia sintered body, comprising a step of firing a zirconia molded body or a zirconia pre-sintered body, wherein: the firing step comprises at least three temperature increase steps including a first temperature increase step (H1), a second temperature increase step (H2), and a third temperature increase step (H3); a temperature increase rate in the first temperature increase step (H1) is defined as HR1, a temperature increase rate in the second temperature increase step (H2) is defined as HR2, and a temperature increase rate in the third temperature increase step (H3) is defined as HR3; HR1 = 50 to 500°C/min, HR2 = 11 to 300°C/min, HR3 = 10 to 299°C/min, HR1 > HR2, and HR2/HR3 > 1 are satisfied; starting temperatures in the temperature increase steps are room temperature to 500°C in H1, 900 to 1250°C in H2, and 1300 to 1550°C in H3; and reaching temperatures in the temperature increase steps are 900 to 1250°C in H1, 1300 to 1550°C in H2, and 1400 to 1650°C in H3.

EP 4 467 125 A2

**Description**

TECHNICAL FIELD

The present invention relates to a method for producing a zirconia sintered body.

BACKGROUND ART

Metals are conventionally commonly used for dental products (for example, typical prostheses such as veneer crowns, tooth crowns, and post crowns, orthodontic products, and dental implant products). However, metals are imperfect in that they lack in aesthetics because of their colors clearly different from natural teeth. Additionally, metal elution can cause allergy. Therefore, to solve the problems attributable to use of metals, ceramic materials such as aluminum oxide (alumina) and zirconium oxide (zirconia) are increasingly used as alternative materials for metals. In particular, there is a growing need for zirconia because of its high strength and relatively high aesthetics and, especially, a recent drop in price.

To produce a dental prosthesis using zirconia, a block-shaped or disc-shaped milling workpiece (workpiece to be milled) pre-sintered at a temperature lower by about 400°C to 700°C than a temperature at which an ideal sintered body can be obtained is curved into a predetermined shape (unsintered zirconia processed body) of a dental prosthesis using a CAD/CAM device. The resulting unsintered processed body is maintained and sintered at a maximum temperature of 1400°C to 1650°C. The total time spent on increasing, maintaining, and decreasing the temperature is generally 6 to 12 hours. As to this total time, recently, there is an increasing demand for short-time firing at dentists, and a firing furnace, as described in Patent Literature 1, allowing firing in a short period of time is also manufactured. However, short-time firing has a problem such as increased lightness, and it is difficult to achieve a color tone or translucency comparable to that achieved by general firing.

CITATION LIST

Patent Literature

Patent Literature 1: JP 2015-531048 A

SUMMARY OF INVENTION

Technical Problem

Patent Literature 1 describes a firing furnace and schedule requirements for firing a ceramic including zirconia in a short period of time. However, the description is not about a schedule for achieving particular, ideal physical properties of a dental zirconia sintered body but about a theoretically achievable firing furnace. There is no description of physical properties, a color tone, etc. demanded of a dental prosthesis obtained from a specific sintered body, and firing requirements for satisfying an aesthetic requirement are not satisfied.

Therefore, the present invention aims to provide a method that is for producing a zirconia sintered body and by which a zirconia molded body or a zirconia pre-sintered body is sintered in a short period of time, the zirconia sintered body being capable of reproducing an aesthetic requirement and strength of an ideal dental prosthesis at the same levels as those of a zirconia sintered body obtained by general firing.

Solution to Problem

The present inventors conducted intensive studies to find a solution to the foregoing issues, and found that a dental prosthesis that is excellent in reproducibility of a color tone (lightness and chroma) and translucency can be produced by using a particular short-time firing schedule for a zirconia molded body or a zirconia pre-sintered body.

The present invention was completed after further studies based on this finding.

Specifically, the present invention includes the following.

[1] A method for producing a zirconia sintered body, comprising a firing step of firing a zirconia molded body or a zirconia pre-sintered body, wherein

the firing step comprises at least three temperature increase steps including a first temperature increase step (H1), a second temperature increase step (H2), and a third temperature increase step (H3),

when a temperature increase rate in the first temperature increase step (H1) is defined as HR1, a temperature increase rate in the second temperature increase step (H2) is defined as HR2, and a temperature increase rate in the third temperature increase step (H3) is defined as HR3,

HR1 = 50 to 500°C/min, HR2 = 11 to 300°C/min, HR3 = 10 to 299°C/min, HR1 > HR2, and HR2/HR3 > 1 are satisfied,

starting temperatures in the temperature increase steps are room temperature to 500°C in H1, 900 to 1250°C in H2, and 1300 to 1550°C in H3, and

reaching temperatures in the temperature increase steps are 900 to 1250°C in H1, 1300 to 1550°C in H2, and 1400 to 1650°C in H3.

[2] The method for producing a zirconia sintered body according to [1], wherein HR2 is 13 to 280°C/min.

[3] The method for producing a zirconia sintered body according to [1] or [2], wherein HR3 is 13 to 250°C/min.

[4] The method for producing a zirconia sintered body according to any one of [1] to [3], wherein HR2/HR3 > 1.5 is satisfied.

[5] The method for producing a zirconia sintered body according to any one of [1] to [4], wherein a maximum firing temperature in the temperature increase steps is 1400 to 1650°C and a duration time in which the maximum firing temperature is maintained is 15 minutes or less.

[6] The method for producing a zirconia sintered body according to any one of [1] to [5], comprising a temperature decrease step of decreasing a temperature from a maximum firing temperature in the temperature increase steps to 1100°C at a temperature decrease rate of 10°C/min or more.

[7] The method for producing a zirconia sintered body according to any one of [1] to [6], wherein a total firing time from a start of a temperature increase in the first temperature increase step (H1) to an end of a duration time in which a maximum firing temperature is maintained is 50 minutes or less in the firing step.

[8] The method for producing a zirconia sintered body according to any one of [1] to [7], wherein 55% or more of the zirconia pre-sintered body is a monoclinic crystal system.

[9] The method for producing a zirconia sintered body according to any one of [1] to [8], wherein the zirconia molded body or the zirconia pre-sintered body includes a stabilizer in an amount of 2 to 8 mol%.

[10] The method for producing a zirconia sintered body according to [9], wherein in the zirconia molded body or the zirconia pre-sintered body, at least a portion of the stabilizer is not dissolved in zirconia as a solid solution.

[11] The method for producing a zirconia sintered body according to [9] or [10], wherein the stabilizer is yttria.

[12] The method for producing a zirconia sintered body according to any one of [1] to [11], wherein the zirconia molded body or the zirconia pre-sintered body has a predetermined shape of a dental product.

[13] The method for producing a zirconia sintered body according to [12], wherein the dental product is a dental prosthesis.

Advantageous Effects of Invention

According to the method for producing a zirconia sintered body according to the present invention, a zirconia molded body or a zirconia pre-sintered body is sintered in a short period of time and a zirconia sintered body capable of reproducing an aesthetic requirement and strength of an ideal dental prosthesis at the same levels as those of a zirconia sintered body obtained by general firing (6- to 12-hour firing) can be produced.

The zirconia sintered body obtained by the method for producing a zirconia sintered body according to the present invention is excellent in color tone reproducibility. According to the method for producing a zirconia sintered body according to the present invention, adequate lightness can be obtained while color formation by a composite oxide included in the zirconia molded body or the zirconia pre-sintered body is promoted.

BRIEF DESCRIPTION OF DRAWINGS

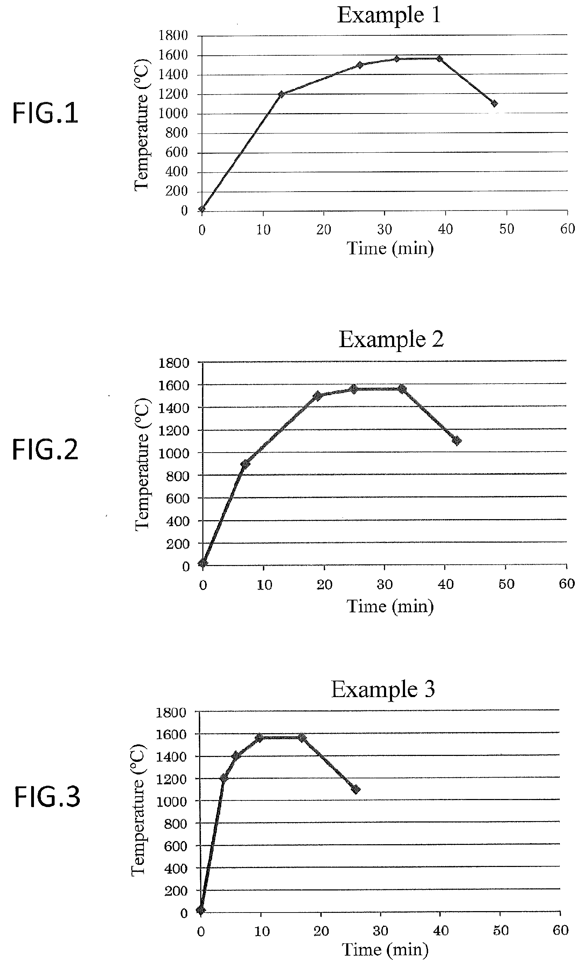

FIG. 1 shows a temperature increase rate in a temperature increase step according to Example 1.
FIG. 2 shows a temperature increase rate in a temperature increase step according to Example 2.
FIG. 3 shows a temperature increase rate in a temperature increase step according to Example 3.

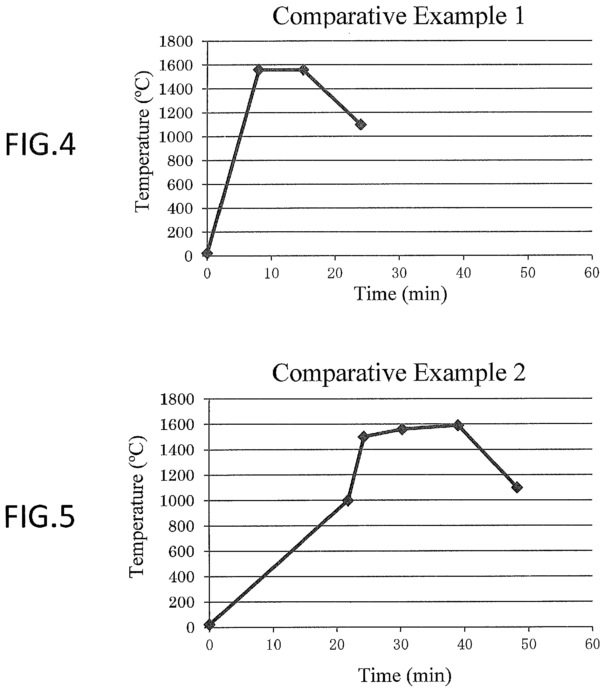

FIG. 4 shows a temperature increase rate in a temperature increase step according to Comparative Example 1.
FIG. 5 shows a temperature increase rate in a temperature increase step according to Comparative Example 2.

DESCRIPTION OF EMBODIMENTS

Hereinafter, the present invention will be described in detail. In the present invention, a firing furnace used for firing is an air furnace. The firing furnace may be a box furnace, a crucible furnace, a tubular furnace, a bottom loading furnace, a continuous furnace, or a rotary kiln, may be a resistive heating furnace, an induction heating furnace, a direct electric furnace, an IH furnace, a high-frequency furnace, or a microwave furnace, may be equipped with, for example, a metal heating element, silicon carbide, molybdenum disilicide, lanthanum chromite, molybdenum, carbon, or tungsten as a heating element, and may be equipped with SiC as a heating element susceptor. The firing furnace may be a combination of two or more thereof. When having a smaller internal volume, the firing furnace including a stage on which a zirconia molded body or a zirconia pre-sintered body having a predetermined shape such as a tooth crown shape is left to stand has better thermal efficiency and can more easily maintain the amount of heat in the furnace during firing.

In the present specification, the upper limits and lower limits of value ranges (ranges of temperature increase rates, temperature decrease rates, firing time, temperatures, rate ratios, amounts of contained components (for example, a stabilizer), etc.) can be combined appropriately.

The method for producing a zirconia sintered body according to the present invention comprises at least three temperature increase steps. Of the temperature increase steps, a first temperature increase step is H1, a second temperature increase step is H2, a third temperature increase step is H3, and the temperature increase steps have different temperature increase rates from each other. The temperature increase steps may include only the three steps or may include an additional temperature increase step.

First Temperature Increase Step (H1)

In the first temperature increase step (H1) of the method for producing a zirconia sintered body according to the present invention, the temperature of a firing furnace at room temperature or having heated to more than room temperature and 500°C or less is sharply increased to a reaching temperature of the first temperature increase step (H1) to heat a zirconia molded body or a zirconia pre-sintered body therein. Before the firing in the first temperature increase step (H1), the zirconia molded body or the zirconia pre-sintered body preferably has a predetermined shape of a dental product. Examples of the dental product include: typical dental prostheses such as veneer crowns, tooth crowns, and post crowns; orthodontic products; and dental implant products. The zirconia molded body or the zirconia pre-sintered body yet to be heated may be processed using a dental CAD/CAM system or may be fabricated by a dental technician, for example, by milling processing.

When fired, the zirconia molded body or the zirconia pre-sintered body yet to be heated may be left to stand directly on a muffle member of a firing furnace, may be left to stand in a furnace using a tray or a stage made of a ceramic or a high-melting-point metal or using a pin, or may be left to stand using a ceramic bead.

A starting temperature in the first temperature increase step (H1) is room temperature to 500°C, preferably room temperature to 400°C, more preferably room temperature to 300°C, even more preferably room temperature to 200°C. The reaching temperature in the first temperature increase step (H1) is 900°C to 1250°C and, in terms of a shorter operation time, preferably 950°C or more, more preferably 1000°C or more, even more preferably 1050°C or more. In terms of better lightness and translucency of the resulting zirconia sintered body, the reaching temperature in the first temperature increase step (H1) is preferably 1230°C or less, more preferably 1220°C or less, even more preferably 1200°C or less.

When a temperature increase rate in the first temperature increase step (H1) is defined as HR1, HR1 is 50°C/min or more and, in terms of a shorter operation time, preferably 60°C/min or more, more preferably 70°C/min or more, even more preferably 80°C/min or more. HR1 is 500°C/min or less, preferably 450°C/min or less, more preferably 400°C/min or less, even more preferably 350°C/min or less. If HR1 exceeded 500°C/min, occurrence of split or crack might be induced during the firing. When the zirconia molded body or the zirconia pre-sintered body used in the first temperature increase step (H1) includes moisture used in processing thereof or a color liquid for coloring, the first temperature increase step (H1) may be started after a drying step at 300°C or less for 1 minute or more and 20 minutes or less, preferably for 5 minutes or more and 15 minutes or less.

Second Temperature Increase Step (H2)

In the method for producing a zirconia sintered body according to the present invention, when a temperature increase rate in the second temperature increase step (H2) is defined as HR2, HR1 > HR2 is satisfied. Because of HR1 > HR2, in combination with another feature, a color difference $\Delta E^*ab$ of the resulting zirconia sintered body can be prevented from becoming too large and a dental product having a good color tone can be obtained. In a suitable embodiment, HR1/HR2 > 1.5 is satisfied, and HR1/HR2 > 2 may be satisfied. HR2 is 11°C/min or more and, in terms of a shorter operation time, preferably 13°C/min or more, more preferably 15°C/min or more, even more preferably 20°C/min or more. HR2 is 300°C/min or less and, in terms of better lightness and translucency of the resulting zirconia sintered body, preferably 280°C/min or less, more preferably 260°C/min or less, even more preferably 250°C/min or less.

A starting temperature in the second temperature increase step (H2) is 900 to 1250°C and, in terms of a shorter operation time, preferably 950°C or more, more preferably 1000°C or more, even more preferably 1050°C or more. In terms of better lightness and translucency of the resulting zirconia sintered body, the starting temperature is preferably 1230°C or less, more preferably 1220°C or less, even more preferably 1200°C or less. A reaching temperature in the second temperature increase step (H2) is 1300 to 1550°C and, in terms of a shorter operation time, preferably 1310°C or more, more preferably 1320°C or more, even more preferably 1350°C or more. In terms of better lightness, translucency, and chroma of the resulting zirconia sintered body and high color formation performance of a composite oxide included in the zirconia molded body or the zirconia pre-sintered body, the reaching temperature in H2 is preferably 1540°C or less, more preferably 1530°C or less, even more preferably 1520°C or less.

Third Temperature Increase Step (H3)

When a temperature increase rate of the third temperature increase step (H3) is defined as HR3, HR2/HR3 > 1 is satisfied, and, in terms of better lightness, translucency, and chroma of the resulting zirconia sintered body and high color formation performance of a composite oxide included in the zirconia molded body or the zirconia pre-sintered body, preferably HR2/HR3 > 1.2, more preferably HR2/HR3 > 1.5, even more preferably HR2/HR3 > 2 is satisfied. HR3 is 10°C/min or more and, in terms of a shorter operation time, preferably 11°C/min or more, more preferably 12°C/min or more, even more preferably 13°C/min or more. In terms of better chroma of the resulting zirconia sintered body and high color formation performance of a composite oxide included in the zirconia molded body or the zirconia pre-sintered body, HR3 is 299°C/min or less, preferably 270°C/min or less, more preferably 250°C/min or less, even more preferably 200°C/min or less.

A starting temperature in the third temperature increase step (H3) is 1300 to 1550°C and, in terms of a shorter operation time, preferably 1310°C or more, more preferably 1320°C or more, even more preferably 1350°C or more. In terms of better lightness, translucency, and chroma of the resulting zirconia sintered body and high color formation performance of a composite oxide included in the zirconia molded body or the zirconia pre-sintered body, the starting temperature in H3 is preferably 1540°C or less, more preferably 1530°C or less, even more preferably 1520°C or less.

A reaching temperature in the third temperature increase step (H3) is 1400 to 1650°C and, in terms of better lightness, translucency, and chroma of the resulting zirconia sintered body and high color formation performance of a composite oxide included in the zirconia molded body or the zirconia pre-sintered body, preferably 1450°C or more, more preferably 1500°C or more, even more preferably 1520°C or more. In terms of a shorter operation time, better lightness, translucency, and chroma of the resulting zirconia sintered body, and high color formation performance of a composite oxide included in the zirconia molded body or the zirconia pre-sintered body, the reaching temperature in the third temperature increase step (H3) is preferably 1630°C or less, more preferably 1620°C or less, even more preferably 1610°C or less. A difference (reaching temperature - starting temperature) between the starting temperature and the reaching temperature in the third temperature increase step (H3) is preferably 30°C or more, more preferably 40°C or more, even more preferably 50°C or more. In one embodiment, a treating time in the third temperature increase step (H3) is preferably shorter than a treating time in the first temperature increase step (H1) in terms of better lightness, translucency, and chroma of the resulting zirconia sintered body and high color formation performance of a composite oxide included in the zirconia molded body or the zirconia pre-sintered body. In another embodiment, the treating time in the third temperature increase step (H3) is preferably shorter than a treating time in the second temperature increase step (H2) in terms of better lightness, translucency, and chroma of the resulting zirconia sintered body and high color formation performance of a composite oxide included in the zirconia molded body or the zirconia pre-sintered body.

The zirconia molded body or the zirconia pre-sintered body used in the method for producing a zirconia sintered body according to the present invention preferably comprises, in addition to zirconia, a stabilizer capable of inhibiting a phase transformation of zirconia. The zirconia molded body or the zirconia pre-sintered body is preferably a zirconia molded body or a zirconia pre-sintered body in which at least a portion of the stabilizer is not dissolved in zirconia as a solid solution. The stabilizer is preferably one capable of forming partially stabilized zirconia.

Examples of the stabilizer include oxides such as calcium oxide (CaO), magnesium oxide (MgO), yttria, cerium oxide ($CeO_2$), scandium oxide ($Sc_2O_3$), niobium oxide ($Nb_2O_3$), lanthanum oxide ($La_2O_3$), erbium oxide ($Er_2O_3$), praseodymium oxide (PreOn), samarium oxide ($Sm_2O_3$), europium oxide ($Eu_2O_3$), and thulium oxide ($Tm_2O_3$). The stabilizer may be used alone, or two or more thereof may be used in combination. The content of the stabilizer in the zirconia pre-sintered body of the present invention and the content of the stabilizer in a sintered body of the zirconia pre-sintered body of the present invention can be measured using a technique, for example, such as inductively coupled plasma (ICP) emission spectral analysis or x-ray fluorescence analysis. The content of the stabilizer in the zirconia pre-sintered body of the present invention and that in a sintered body of the zirconia pre-sintered body of the present invention are preferably 0.1 to 18 mol%, more preferably 1 to 15 mol%, even more preferably 2 to 8 mol% relative to the total mole of the zirconia and the stabilizer. The zirconia molded body or the zirconia pre-sintered body preferably includes yttria as the stabilizer from the viewpoint of the strength and translucency of the resulting zirconia sintered body. The yttria content is preferably 3 mol% or more, more preferably 3.5 mol% or more, even more preferably 3.8 mol% or more, particularly preferably 4.0 mol% or more relative to the total mole of zirconia and yttria. The translucency of the zirconia sintered body can increase with a yttria content of 3 mol% or more. The yttria content is preferably 7.5 mol% or less, more preferably 7.0 mol% or less, even more preferably 6.5 mol% or less, particularly preferably 6.0 mol% or less relative to the total mole of zirconia and yttria. Decrease of the strength of the resulting zirconia sintered body can be reduced with a yttria content of 7.5 mol% or less.

In the zirconia molded body or the zirconia pre-sintered body of the present invention, it is preferred that at least a portion of the stabilizer be undissolved in zirconia as a solid solution. Whether or not a portion of the stabilizer is dissolved in zirconia as a solid solution can be confirmed by an XRD pattern, for example. The presence of a peak derived from the stabilizer in an XRD pattern of the zirconia pre-sintered body means that the zirconia molded body or the zirconia pre-sintered body is containing a stabilizer that is not dissolved in zirconia as a solid solution. A peak derived from the stabilizer is basically not observable in an XRD pattern when the stabilizer has fully dissolved as a solid solution. It is, however, possible, depending on the crystal state or other conditions of the stabilizer, that the stabilizer may not be dissolved in zirconia as a solid solution even when the stabilizer does not produce a peak in the XRD pattern. The stabilizer can be thought of having dissolved in zirconia as a solid solution for the most part, basically fully, when zirconia is predominantly a tetragonal and/or cubic system, and there is no peak attributed to the stabilizer in the XRD pattern. In the zirconia molded body or the zirconia pre-sintered body of the present invention, it is not required to fully dissolve the stabilizer in zirconia as a solid solution. In the present invention, "to dissolve the stabilizer as a solid solution" means that, for example, the elements (atoms) contained in the stabilizer are dissolved in zirconia as a solid solution.

In the zirconia molded body or the zirconia pre-sintered body of the present invention, the percentage presence $f_y$ of yttria not dissolved in zirconia as a solid solution (hereinafter, referred to also as "undissolved yttria") can be calculated from the mathematical expression (1) below. The percentage presence $f_y$ of undissolved yttria is preferably more than 0%, more preferably 1% or more, even more preferably 2% or more, particularly preferably 3% or more. The upper limit of the percentage presence $f_y$ of undissolved yttria may be, for example, 15% or less. However, suitably, the upper limit of the percentage presence $f_y$ of undissolved yttria depends on the yttria content in the zirconia molded body or the zirconia pre-sintered body. The percentage presence $f_y$ may be 7% or less for a yttria content of 3 mol% or more and less than 4.5 mol%. The percentage presence $f_y$ may be 11% or less for a yttria content of 4.5 mol% or more and less than 5.8 mol%. The percentage presence $f_y$ may be 15% or less for a yttria content of 5.8 mol% or more and 7.5 mol% or less.

In the zirconia molded body or the zirconia pre-sintered body of the present invention, the percentage presence $f_y$ is preferably 0.5% or more, more preferably 1.0% or more, even more preferably 2.0% or more for a yttria content of 3 mol% or more and less than 4.5 mol%. The percentage presence $f_y$ of undissolved yttria is preferably 1% or more, more preferably 2% or more, even more preferably 3% or more for a yttria content of 4.5 mol% or more and less than 5.8 mol%. The percentage presence $f_y$ is preferably 2% or more, more preferably 3% or more, even more preferably 4% or more for a yttria content of 5.8 mol% or more and 7.5 mol% or less. In the zirconia pre-sintered body of the present invention, the ratio $f_m/f_y$ is preferably 20 to 200, more preferably 25 to 100, even more preferably 30 to 60 for a yttria content of 3 mol% or more and less than 4.5 mol%. The ratio $f_m/f_y$ is preferably 5 to 45, more preferably 10 to 40, even more preferably 15 to 35 for a yttria content of 4.5 mol% or more and less than 5.8 mol%. The ratio $f_m/f_y$ is preferably 2 to 40, more preferably 5 to 35, even more preferably 10 to 30 for a yttria content of 5.8 mol% or more and 7.5 mol% or less. As later described, $f_m$ refers to a fraction of a monoclinic crystal system in zirconia calculated by a mathematical expression (2).

[Math. 1]

$$f_y (\%) = \frac{I_y(111)}{I_y(111) + I_m(111) + I_m(11-1) + I_t(111) + I_c(111)} \times 100 \qquad (1)$$

In the mathematical expression (1), $I_y(111)$ represents the peak intensity of the

(111) plane of yttria in the vicinity of $2\theta = 29°$ in an XRD pattern using CuK radiation. $I_m(111)$ and $I_m(11-1)$ represent the peak intensities of the (111) plane and (11-1) plane, respectively, of the monoclinic crystal system of zirconia. $I_t(111)$ represents the peak intensity of the (111) plane of a tetragonal crystal system of zirconia. $I_c(111)$ represents the peak intensity of the (111) plane of a cubic crystal system of zirconia.

The mathematical expression (1) is also applicable to calculations of the percentage presence of undissolved stabilizers as a solid solution other than yttria by substituting other peaks for $I_y(111)$.

In the zirconia pre-sintered body of the present invention, the zirconia is preferably predominantly monoclinic. In the present invention, "predominantly monoclinic" means that the fraction $f_m$ of the monoclinic crystal system of zirconia is at least 50% of the total amount of all crystal systems of zirconia (the monoclinic system, the tetragonal system, and the cubic system) as calculated from the mathematical expression (2) below. In the zirconia pre-sintered body of the present invention, the fraction $f_m$ of the monoclinic crystal system in zirconia calculated from the mathematical expression (2) below is preferably 55% or more, more preferably 60% or more, even more preferably 70% or more, yet more preferably 75% or more, particularly preferably 80% or more, more particularly preferably 85% or more, most preferably 90% or more relative to the total amount of the monoclinic, tetragonal, and cubic crystal systems. The fraction $f_m$ of the monoclinic crystal system can be calculated from the mathematical expression (2) below, using peaks in an X-ray diffraction (XRD) pattern by CuK radiation. It is to be noted that the predominant crystal system in the zirconia pre-sintered body has possible contribution to the increased contraction temperature and the reduced firing time.

In the zirconia pre-sintered body of the present invention, the peaks of the tetragonal and cubic crystal systems may be essentially undetectable. That is, the monoclinic crystal system may have a fraction $f_m$ of 100%.

[Math. 2]

$$f_m(\%) = \frac{l_m(111) + l_m(11-1)}{l_m(111) + l_m(11-1) + l_t(111) + l_c(111)} \times 100 \quad (2)$$

In the mathematical expression (2), $I_m(111)$ and $I_m(11\text{-}1)$ represent the peak intensities of the (111) plane and (11-1) plane, respectively, of the monoclinic crystal system of zirconia. It(111) represents the peak intensity of the (111) plane of the tetragonal crystal system of zirconia. $I_c(111)$ represents the peak intensity of the (111) plane of the cubic crystal system of zirconia.

The zirconia molded body or the zirconia pre-sintered body may optionally include an additive. Examples of the additive include binders, colorants (including pigments, complex pigments, and fluorescent agents), alumina ($Al_2O_3$), titanium oxide ($TiO_2$), and silica ($SiO_2$). The additive may be used alone, or a mixture of two or more thereof may be used.

Examples of the binder include polyvinyl alcohol, methylcellulose, carboxymethylcellulose, acrylic binders, wax binders (such as paraffin wax), polyvinyl butyral, polymethyl methacrylate, ethyl cellulose, polyethylene, polypropylene, ethylenevinyl acetate copolymer, polystyrene, atactic polypropylene, methacrylic resin, and stearic acid.

Examples of the pigments include oxides of at least one element selected from the group consisting of Ti, V, Cr, Mn, Fe, Co, Ni, Zn, Y, Zr, Sn, Sb, Bi, Ce, Pr, Sm, Eu, Gd, Tb, and Er (specifically, for example, NiO, $Cr_2O_3$), preferably oxides of at least one element selected from the group consisting of Ti, V, Cr, Mn, Fe, Co, Ni, Zn, Y, Zr, Sn, Sb, Bi, Ce, Pr, Sm, Eu, Gd, and Tb, more preferably oxides of at least one element selected from the group consisting of Ti, V, Cr, Mn, Fe, Co, Ni, Zn, Y, Zr, Sn, Sb, Bi, Ce, Sm, Eu, Gd, and Tb. The zirconia molded body or the zirconia pre-sintered body of the present invention may be one that does not comprise erbium oxide ($Er_2O_3$). Examples of the complex pigments include composite oxides such as $(Zr,V)O_2$, $Fe(Fe,Cr)_2O_4$, $(Ni,Co,Fe)(Fe,Cr)_2O_4 \times ZrSiO_4$, and $(Co,Zn)Al_2O_4$. Examples of the fluorescent agents include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

A method for producing the zirconia molded body used in the present invention is not particularly limited, and is, for example, a production method including a step of press-molding a mixed powder composed of zirconia and the stabilizer at a pressure of 175 MPa or more to obtain a zirconia molded body. The bulk density of the resulting zirconia molded body (and a zirconia sintered body to be obtained therefrom) can be increased by press molding at the above pressure, irrespective of the thickness. In the present specification, the above pressure, 175 MPa or more, is the maximum pressure in the press molding.

A method for producing the zirconia pre-sintered body used in the present invention is not particularly limited, and is, for example, a production method in which a zirconia molded body made from a raw material powder containing zirconia particles and a stabilizer is fired (i.e., pre-sintered) at a temperature at which the zirconia particles are not sintered. The zirconia molded body is as described above. An example of the method for producing the zirconia pre-sintered body of the present invention is described below. First, a raw material powder of a zirconia molded body is produced. A monoclinic zirconia powder and a stabilizer powder (for example, a yttria powder) are used to make a mixture of a desired stabilizer (for example, yttria) content. The mixture is added to water to prepare a slurry, and pulverized and mixed wet with a ball mill until the desired particle size is achieved. After pulverization, the slurry is dried to granulate, using a spray dryer. The resulting powder is then fired into a primary powder at a temperature (for example, 800 to 1200°C) at which the zirconia particles are not sintered. The pigment may be added to the primary powder. The primary powder is added to water to prepare a slurry, and pulverized and mixed wet with a ball mill until the desired particle size is achieved. After pulverization, an additive, such as a binder, is optionally added to the slurry, and the slurry is dried with a spray dryer to produce a mixed powder (secondary powder). The secondary powder is charged into a predetermined die. A top surface thereof is leveled, and an upper die is set on the flat top surface. The secondary powder is press-molded using a uniaxial pressing machine to obtain a zirconia molded body. As described above, the pressure at which the above mixed powder is press-molded is preferably, 175 MPa or more. The resulting zirconia molded body may or may not be subjected to cold isostatic press (CIP) molding.

The zirconia pre-sintered body may have a multilayer structure. In order to produce the zirconia pre-sintered body having a multilayer structure, the primary powder may be divided into at least two portions (suitably four portions) in the above production method, so that a zirconia molded body having a multilayer structure is formed.

Next, the zirconia molded body obtained in the above manner is pre-sintered to obtain a zirconia molded body. The pre-sintering temperature is, for example, preferably 800°C or more, more preferably 900°C or more, even more preferably 950°C or more. For improved dimensional accuracy, the pre-sintering temperature is, for example, preferably 1200°C or less, more preferably 1150°C or less, even more preferably 1100°C or less. This is because pre-sintering at the pre-sintering temperature falling in this range should not drive dissolution of the stabilizer as a solid solution.

The zirconia pre-sintered body used in the present invention may be a commercially-available product. Examples of the commercially-available product include zirconia pre-sintered bodies, such as "NORITAKE KATANA (registered trademark) zirconia" (model numbers: disc UTML, disc STML, disc ML, disc HT, disc LT) (manufactured by Kuraray Noritake Dental Inc.), including yttria as the stabilizer. In the method for producing a zirconia sintered body according to the present invention, it is preferred that the above commercially-available zirconia pre-sintered body be used after formed into a predetermined shape of a dental product by milling processing.

In the method for producing a zirconia sintered body according to the present invention, each of the temperature increase rates in the temperature increase steps may be a constant rate or may be a multistage rate in which the rate changes in the middle of the step, as long as the starting temperatures, the reaching temperatures, the ranges of the temperature increase rates, and the relations HR1> HR2 and HR2/HR3 > 1 in the temperature increase steps are satisfied. For example, in one embodiment, in the second temperature increase step, the temperature may increase from the starting temperature at 150°C/min for 30 seconds and then at 80°C/min. In another embodiment, in the third temperature increase step, the temperature may increase from the starting temperature at 60°C/min for 30 seconds and then at 20°C/min.

Maintaining Step

In the method for producing a zirconia sintered body according to the present invention, a duration time in which a maximum firing temperature is maintained is preferably 15 minutes or less, and, in terms of a shorter operation time and excellent strength of the resulting zirconia sintered body, more preferably 1 to 15 minutes, even more preferably 5 to 15 minutes. The maximum firing temperature is, in a suitable embodiment, 1400 to 1650°C and, in terms of better lightness, translucency, and chroma of the resulting zirconia sintered body and high color formation performance of the composite oxide included in the zirconia molded body or the zirconia pre-sintered body, preferably 1450°C or more, more preferably 1500°C or more, even more preferably 1520°C or more. In terms of a shorter operation time, better lightness, translucency, and chroma of the resulting zirconia sintered body, and high color formation performance of the composite oxide included in the zirconia molded body or the zirconia pre-sintered body, the maximum firing temperature is preferably 1630°C or less, more preferably 1620°C or less, even more preferably 1610°C or less. The maintaining step preferably comes just after the third temperature increase step, but there may be another temperature increase step between the third temperature increase step and the maintaining step, provided that the present invention can exhibit its effects. In an embodiment including no other temperature increase step than the above steps, the reaching temperature in H3 is the maximum firing temperature.

In a firing step, a total firing time from a start of a temperature increase in the first temperature increase step to an end of the duration time in which the maximum firing temperature is maintained is preferably 50 minutes or less, more preferably 45 minutes or less, even more preferably 40 minutes or less in terms of a shorter operation time.

Cooling Step

The method for producing a zirconia sintered body according to the present invention preferably comprises a cooling step after the maximum firing temperature is kept for the predetermined period of time. In the cooling step, a temperature decrease rate at which the temperature decreases from the maximum firing temperature at which a zirconia sintered body is obtained to 1100°C is preferably 10°C/min or more, more preferably 30°C/min or more, even more preferably 50°C/min or more. The temperature is decreased by cooling with outdoor air, cooling with water, cooling with air, slow cooling, or leaving the sintered body to cool down, or by any combination of these techniques. The reaching temperature in the cooling step varies depending on, for example, the type and performance of a firing furnace, and may be 1300°C, 1050°C, or 1000°C.

The color difference ΔE*ab of a zirconia sintered body obtained by the production method according to the present invention is preferably 2.7 or less, more preferably 2.0 or less, even more preferably 1.6 or less, particularly preferably 0.8 or less because of suitability for a dental product. The color difference ΔE*ab is obtained by comparison with a color tone of a zirconia sintered body having undergone general firing (total firing time: 6 to 12 hours). The method employed for color tone evaluation is as described in EXAMPLES below.

A difference between a lightness index L* of a zirconia sintered body obtained by the production method according to the present invention and a lightness index L* of a zirconia sintered body having undergone general firing (total firing time: 6 to 12 hours) is preferably 2.0 or less, more preferably 1.5 or less, even more preferably 1.0 or less because of suitability for a dental product. L*, a*, and b* of a zirconia sintered body obtained by the production method according to the present invention can be selected and determined according to an intended section, such as a cervical portion (tooth cervix) or an incisal portion (incisal edge).

The present invention encompasses combinations of the foregoing features, provided that such combinations made in various forms within the technical idea of the present invention can produce the effects of the present invention.

EXAMPLES

Next, the present invention will be described in greater detail by way of Examples. It should be noted that the present invention is in no way limited by the following Examples, and various changes may be made by a person with ordinary skill in the art within the technical idea of the present invention.

Example 1

Ten samples having the shape of a 1.7 mm × 5.2 mm × 20.2 mm rectangular parallelepiped were fabricated by carving a disc-shaped zirconia work, "NORITAKE KATANA (registered trademark) zirconia" STMLA1 color (manufactured by KURARAY NORITAKE DENTAL INC.), using DWX51D (manufactured by Roland D.G). The samples were fired according to firing schedule conditions shown in Table 1 using Programat (registered trademark) CS4 (manufactured by Ivoclar Vivadent K.K.) as a firing furnace. The total firing time from the start of a temperature increase in the first temperature increase step to the end of the duration time in which the maximum firing temperature is maintained was 50 minutes or less for the firing. Zirconia sintered bodies were thus obtained.

Three-Point Flexural Strength

The 10 zirconia sintered bodies obtained by the firing were ground using a rotary grinder and #1000 abrasive paper into sintered body samples having the shape of a 1.2 mm × 4 mm × 16 mm rectangular parallelepiped. The three-point flexural strength of each of the sintered body samples was measured under conditions specified in ISO 6872: 2015, i.e., at a crosshead speed of 0.5 mm/min and a distance between supports (span) of 14 mm. Additionally, to check the three-point flexural strength of a sintered body sample fired according to a conventional firing schedule, sintered body samples fired using NORITAKE KATANA (registered trademark) F1-N (manufactured by KURARAY NORITAKE DENTAL INC.) as a firing furnace according to a firing schedule shown in Table 6 were measured for the three-point flexural strength in the above manner. As shown in Table 7, the average three-point flexural strength of the sintered body samples of Example 1 was comparable to that of the sintered body samples fired according to the conventional firing schedule and was confirmed to have strength sufficient for dental use.

[Table 1]

| Time (min) | Temperature (°C) | Temperature increase/ decrease rate (°C/min) |
|---|---|---|
| 0 | 25 | - |
| 13 | 1200 | 90 |
| 26 | 1500 | 23 |
| 32 | 1560 | 10 |
| 39 | 1560 | Maintained |
| 48 | 1100 | -50 |

Measurement of Lightness, Chroma, and Color Difference

Next, a disc-shaped zirconia work as used for the measurement of three-point flexural strength was carved into the shape of a front tooth crown using DWX51D. Thus-fabricated samples in the same shape were fired according to the conventional firing schedule (Table 6) using NORITAKE KATANA (registered trademark) F1-N as a firing furnace or according to the firing schedule described in Table 1 using Programat (registered trademark) CS4 as a firing furnace.

After the firing, the sintered body samples were subjected to color measurement using a dental color measurement device (manufacture by Olympus Corporation, a 7-band LED light source, "Crystaleye" (manufacture by Olympus Corporation)) to evaluate the lightness, chroma, and color difference ΔE*ab in a L*a*b* color system (JIS Z 8781-4: 2013, Color Measurements - Section 4: CIE 1976 L*a*b* color space) (n = 5). Table 8 shows averages of n = 5 as the evaluation results. The term "color difference ΔE*ab" refers to a color difference ΔE (ΔE*ab) determined for two samples by the following equation (3) using a lightness index L* and chromatic coordinates a* and b* in a CIE 1976 L*a*b* color space, the two samples being one of the sintered body samples ($L_1^*$, $a_1^*$, $b_1^*$) obtained according to the firing schedule shown in Table 6 and one of the sintered body samples ($L_2^*$, $a_2^*$, $b_2^*$) obtained according to the firing schedule shown in Table 1.
[Math. 3]

$$\Delta E^* = \{ (L_1^* - L_2^*)^2 + (a_1^* - a_2^*)^2 + (b_1^* - b_2^*)^2 \}^{1/2} \quad (3)$$

As shown in Table 8, it was confirmed that for the sintered body samples fired according to the firing schedule shown in Table 1, the average of the color differences ΔE*ab from the sintered body samples fired according to the conventional firing schedule shown in Table 6 was 2.7 or less.

Example 2

The three-point flexural strength and color difference ΔE*ab were measured in the same manner as in Example 1, except that the firing schedule was changed to a schedule shown in Table 2. As shown in Table 7, the three-point flexural strength of Example 2 was comparable to that of the sintered body sample fired according to the conventional firing schedule shown in Table 6 and was confirmed to have strength sufficient for dental use. As shown in Table 8, it was confirmed that for the sintered body samples fired according to the firing schedule shown in Table 2, the average of the color differences ΔE*ab from the sintered body samples fired according to the conventional firing schedule shown in Table 6 was 2.7 or less.

[Table 2]

| Time (min) | Temperature (°C) | Temperature increase/ decrease rate (°C/min) |
|---|---|---|
| 0 | 25 | - |
| 7 | 900 | 130 |
| 19 | 1500 | 50 |
| 25 | 1560 | 10 |
| 33 | 1560 | Maintained |
| 42 | 1100 | -50 |

Example 3

The three-point flexural strength and color difference ΔE*ab were measured in the same manner as in Example 1, except that SINTERMAT 1600 (manufactured by The Dental Solution, Inc.) was used as a firing furnace and the firing schedule was changed to a firing schedule shown in Table 3. As shown in Table 7, the three-point flexural strength of Example 3 was comparable to that of the sintered body sample fired according to the conventional firing schedule shown in Table 6 and was confirmed to have strength sufficient for dental use. As shown in Table 8, it was confirmed that for the sintered body samples fired according to the firing schedule shown in Table 3, the average of the color differences ΔE*ab from the sintered body samples fired according to the conventional firing schedule shown in Table 6 was 2.7 or less.

[Table 3]

| Time (min) | Temperature (°C) | Temperature increase/ decrease rate (°C/min) |
|---|---|---|
| 0 | 25 | - |
| 4 | 1200 | 300 |
| 6 | 1400 | 100 |
| 10 | 1565 | 40 |

(continued)

| Time (min) | Temperature (°C) | Temperature increase/ decrease rate (°C/min) |
|---|---|---|
| 17 | 1565 | Maintained |
| 26 | 1100 | -50 |

Comparative Examples 1 and 2

The three-point flexural strength and color difference ΔE*ab were measured in the same manner as in Example 1, except that SINTERMAT 1600 (manufactured by The Dental Solution, Inc.) was used as a firing furnace and the firing schedule was changed to firing schedules shown in Tables 4 and 5. As shown in Table 7, the three-point flexural strengths of Comparative Examples were comparable to that of the sintered body sample fired according to the conventional firing schedule shown in Table 6 and were confirmed to have strengths sufficient for dental use. However, as shown in Table 8, for the sintered body samples fired according to the firing schedules shown in Table 4 (Comparative Example 1) and Table 5 (Comparative Example 2), the averages of the color differences ΔE*ab from the sintered body samples fired according to the conventional firing schedule shown in Table 6 exceeded 2.7, and a color tone comparable to the sintered body samples obtained according to the conventional firing schedule was not obtained. In addition, the zirconia sintered bodies of Comparative Examples 1 and 2 had increased lightness.

[Table 4]

| Time (min) | Temperature (°C) | Temperature increase/ decrease rate (°C/min) |
|---|---|---|
| 0 | 25 | - |
| 8 | 1560 | 200 |
| 15 | 1560 | Maintained |
| 24 | 1100 | -50 |

[Table 5]

| Time (min) | Temperature (°C) | Temperature increase/ decrease rate (°C/min) |
|---|---|---|
| 0 | 25 | - |
| 22 | 900 | 45 |
| 24 | 1500 | 200 |
| 30 | 1560 | 10 |
| 39 | 1560 | Maintained |
| 48 | 1100 | -50 |

[Table 6]

| Time (min) | Temperature (°C) | Temperature increase/ decrease rate (°C/min) |
|---|---|---|
| 0 | 25 | - |
| 148 | 1550 | 10 |
| 268 | 1550 | Maintained |
| 373 | 500 | -10 |

[Table 7]

| Firing condition | Conventional method (Table 6) | Example 1 | Example 2 | Example 3 | Comparative Example 1 | Comparative Example 2 |
|---|---|---|---|---|---|---|
| Flexural strength (MPa) | 780 | 791 | 775 | 793 | 771 | 785 |

[Table 8]

| Firing condition | | Conventional method (Table 6) | Example 1 | Example 2 | Example 3 | Comparative Example 1 | Comparative Example 2 |
|---|---|---|---|---|---|---|---|
| Shade | | A1 | | | | | |
| Cervical | L* | 69.64 | 68.75 | 70.26 | 70.13 | 73.36 | 72.62 |
| | a* | -1.74 | -1.90 | -2.08 | -1.75 | -1.08 | -1.35 |
| | b* | 13.37 | 14.05 | 14.12 | 13.14 | 14.44 | 13.80 |
| ΔE*ab | | | 1.13 | 1.03 | 0.54 | 3.92 | 3.04 |
| Body | L* | 73.51 | 73.04 | 74.38 | 74.17 | 75.71 | 75.21 |
| | a* | -2.28 | -2.47 | -2.67 | -2.37 | -3.23 | -3.05 |
| | b* | 12.14 | 12.28 | 12.26 | 13.44 | 14.58 | 14.23 |
| ΔE*ab | | | 0.53 | 0.96 | 1.46 | 3.43 | 2.80 |
| Incisal | L* | 74.09 | 74.05 | 74.46 | 73.46 | 76.30 | 75.89 |
| | a* | -2.73 | -2.77 | -2.10 | -2.08 | -3.51 | -3.24 |
| | b* | 8.59 | 9.00 | 8.95 | 8.79 | 11.23 | 10.78 |
| ΔE*ab ΔE* | | | 0.42 | 0.81 | 0.93 | 3.53 | 2.88 0.42 |

Furthermore, it has been visually confirmed that in spite of the short total firing time, namely, 50 minutes or less, the zirconia sintered bodies of Examples 1 to 3 had adequate translucency comparable to that of the zirconia sintered body obtained by the general firing (total firing time: 6 to 12 hours). The above results have confirmed that a zirconia sintered body obtained by the production method according to the present invention has high strength, reduces an increase in lightness, has adequate translucency, has a good color tone, and is suitable as a dental product (for example, a dental prosthesis).

INDUSTRIAL APPLICABILITY

The method for producing a zirconia sintered body according to the present invention is useful in producing a dental product (such as a dental prosthesis) because the zirconia molded body or the zirconia pre-sintered body is sintered in a short period of time and the resulting zirconia sintered body can reproduce aesthetic requirements (a color tone and translucency) and strength of an ideal dental prosthesis at the same levels as those of a zirconia sintered body obtained by general firing (total firing time: 6 to 12 hours). The method for producing a zirconia sintered body according to the present invention is useful particularly in producing a dental prosthesis such as a post crown because the resulting zirconia sintered body has a good color tone and has translucency as of an incisal edge of a natural front tooth.

Further aspects and embodiments of the present disclosure are set out in the numbered clauses below.

1. A method for producing a zirconia sintered body, comprising a firing step of firing a zirconia molded body or a zirconia pre-sintered body, wherein

the firing step comprises at least three temperature increase steps including a first temperature increase step (H1), a second temperature increase step (H2), and a third temperature increase step (H3),
when a temperature increase rate in the first temperature increase step (H1) is defined as HR1, a temperature increase rate in the second temperature increase step (H2) is defined as HR2, and a temperature increase rate in the third temperature increase step (H3) is defined as HR3,

HR1 = 50 to 500°C/min, HR2 = 11 to 300°C/min, HR3 = 10 to 299°C/min, HR1 > HR2, and HR2/HR3 > 1 are satisfied,

starting temperatures in the temperature increase steps are room temperature to 500°C in H1, 900 to 1250°C in H2, and 1300 to 1550°C in H3, and

reaching temperatures in the temperature increase steps are 900 to 1250°C in H1, 1300 to 1550°C in H2, and 1400 to 1650°C in H3.

2. The method for producing a zirconia sintered body according to clause 1, wherein HR2 is 13 to 280°C/min.

3. The method for producing a zirconia sintered body according to clause 1 or 2, wherein HR3 is 13 to 250°C/min.

4. The method for producing a zirconia sintered body according to any one of clauses 1 to 3, wherein HR2/HR3 > 1.5 is satisfied.

5. The method for producing a zirconia sintered body according to any one of clauses 1 to 4, wherein a maximum firing temperature in the temperature increase steps is 1400 to 1650°C and a duration time in which the maximum firing temperature is maintained is 15 minutes or less.

6. The method for producing a zirconia sintered body according to any one of clauses 1 to 5, comprising a temperature decrease step of decreasing a temperature from a maximum firing temperature in the temperature increase steps to 1100°C at a temperature decrease rate of 10°C/min or more.

7. The method for producing a zirconia sintered body according to any one of clauses 1 to 6, wherein a total firing time from a start of a temperature increase in the first temperature increase step (H1) to an end of a duration time in which a maximum firing temperature is maintained is 50 minutes or less in the firing step.

8. The method for producing a zirconia sintered body according to any one of clauses 1 to 7, wherein 55% or more of the zirconia pre-sintered body is a monoclinic crystal system.

9. The method for producing a zirconia sintered body according to any one of clauses 1 to 8, wherein the zirconia molded body or the zirconia pre-sintered body includes a stabilizer in an amount of 2 to 8 mol%.

10. The method for producing a zirconia sintered body according to clause 9, wherein in the zirconia molded body or the zirconia pre-sintered body, at least a portion of the stabilizer is not dissolved in zirconia as a solid solution.

11. The method for producing a zirconia sintered body according to clause 9 or 10, wherein the stabilizer is yttria.

12. The method for producing a zirconia sintered body according to any one of clauses 1 to 11, wherein the zirconia molded body or the zirconia pre-sintered body has a predetermined shape of a dental product.

13. The method for producing a zirconia sintered body according to clause 12, wherein the dental product is a dental prosthesis.

**Claims**

1. A method for producing a zirconia sintered body, comprising a firing step of firing a zirconia molded body or a zirconia pre-sintered body, wherein

the firing step comprises at least three temperature increase steps including a first temperature increase step (H1), a second temperature increase step (H2), and a third temperature increase step (H3),

when a temperature increase rate in the first temperature increase step (H1) is defined as HR1, a temperature increase rate in the second temperature increase step (H2) is defined as HR2, and a temperature increase rate in the third temperature increase step (H3) is defined as HR3,

HR1 = 50 to 500°C/min, HR2 = 11 to 300°C/min, HR3 = 10 to 299°C/min, HR1 > HR2, and HR2/HR3 > 1 are satisfied,

starting temperatures in the temperature increase steps are room temperature to 500°C in H1, 900 to 1250°C in H2, and 1300 to 1550°C in H3, and

reaching temperatures in the temperature increase steps are 900 to 1250°C in H1, 1300 to 1550°C in H2, and 1400 to 1650°C in H3.

2. The method for producing a zirconia sintered body according to claim 1, wherein HR2 is 13 to 280°C/min.

3. The method for producing a zirconia sintered body according to claim 1 or 2, wherein HR3 is 13 to 250°C/min.

4. The method for producing a zirconia sintered body according to any one of claims 1 to 3, wherein HR2/HR3 > 1.5 is satisfied.

5. The method for producing a zirconia sintered body according to any one of claims 1 to 4, wherein a maximum firing temperature in the temperature increase steps is 1400 to 1650°C and a duration time in which the maximum firing temperature is maintained is 15 minutes or less.

6. The method for producing a zirconia sintered body according to any one of claims 1 to 5, comprising a temperature decrease step of decreasing a temperature from a maximum firing temperature in the temperature increase steps to 1100°C at a temperature decrease rate of 10°C/min or more.

7. The method for producing a zirconia sintered body according to any one of claims 1 to 6, wherein a total firing time from a start of a temperature increase in the first temperature increase step (H1) to an end of a duration time in which a maximum firing temperature is maintained is 50 minutes or less in the firing step.

8. The method for producing a zirconia sintered body according to any one of claims 1 to 7, wherein 55% or more of the zirconia pre-sintered body is a monoclinic crystal system.

9. The method for producing a zirconia sintered body according to any one of claims 1 to 8, wherein the zirconia molded body or the zirconia pre-sintered body includes a stabilizer in an amount of 2 to 8 mol%.

10. The method for producing a zirconia sintered body according to claim 9, wherein in the zirconia molded body or the zirconia pre-sintered body, at least a portion of the stabilizer is not dissolved in zirconia as a solid solution.

11. The method for producing a zirconia sintered body according to claim 9 or 10, wherein the stabilizer is yttria.

12. The method for producing a zirconia sintered body according to any one of claims 1 to 11, wherein the zirconia molded body or the zirconia pre-sintered body has a predetermined shape of a dental product.

13. The method for producing a zirconia sintered body according to claim 12, wherein the dental product is a dental prosthesis.

FIG.1

Example 1

FIG.2

Example 2

FIG.3

Example 3

## Comparative Example 1

FIG.4

## Comparative Example 2

FIG.5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015531048 A